# EUROPEAN PATENT SPECIFICATION

(11) **EP 2 097 120 B1**
(45) Date of publication and mention of the grant of the patent: **03.08.2011**
(21) Application number: 07827386.9
(22) Date of filing: 14.11.2007
(51) Int. Cl.: A61L 31/14, A61L 31/02, A61B 17/86

(54) **PEDICLE SCREW SURFACE TREATMENT FOR IMPROVING BONE-IMPLANT INTERFACE**
PEDIKELSCHRAUBEN-OBERFLÄCHENBEHANDLUNG ZUR VERBESSERUNG DER SCHNITTSTELLE ZWISCHEN KNOCHEN UND IMPLANTAT
TRAITEMENT DE SURFACE D'UNE VIS PÉDICULAIRE POUR AMÉLIORATION DE L'INTERFACE D'UN IMPLANT OSSEUX

(30) Priority: 15.11.2006 US 865876 P
(43) Date of publication of application: 09.09.2009
(73) Proprietor: Impliant Ltd., 42504 Ramat Poleg (IL)
(72) Inventor: ARNIN, Uri, 36031 Kiryat Tivon (IL)
(74) Representative: Lecomte, Didier
(86) International application number: PCT/IL2007/001413
(87) International publication number: WO 2008/059505

(56) References cited:
- WO-A-2004/089245
- WO-A-2005/074530
- US-A1- 2005 131 409
- PIATTELLI MAURIZIO ET AL: "Bone response to machined and resorbable blast material titanium implants: an experimental study in rabbits." THE JOURNAL OF ORAL IMPLANTOLOGY 2002, vol. 28, no. 1, 2002, pages 2-8, XP002523238 ISSN: 0160-6972

## Description

### FIELD OF THE INVENTION

The present invention is related generally to surface treatment of pedicle screws to improve a bone-implant interface.

### BACKGROUND OF THE INVENTION

One main concern when using pedicle screw based implants is screw loosening. The loads acting on the human spine during normal daily life transfer a variety of forces and moments to the pedicle screws that may lead to micro-motions in the bone-screw interface and delay or prevent the fixation of the screw to the bone.

In the general orthopedic field, one of the most common ways to improve osseointegration is by the addition of HA (Hydroxyapatite) coating, which is in clinical use since 1986. The short term results of the coating showed high bone to implant contact percentage, but over the mid and long terms two main disadvantages of the HA coating were presented. It was found that the first generation of HA tends to resorb and delaminate, leading to poor attachment between the implant and the bone. Today, a second generation of HA coating is used, but there is still a great lack of certainty in terms of long-term results. A third generation of HA nano-coating is now in research and development.

The international publication WO2005/0745 discloses an implantable device composed of a biocompatible material having an enhanced surface topography that has an implanted calcium ion concentration and a method of making the same. In particular it is disclosed a method for surface treatment of a bone implant by blasting the surface with calcium phosphate.

### SUMMARY OF THE INVENTION

The present invention seeks to provide a surface treatment of pedicle screws so as to improve the interface between bone and implant, as is described more in detail hereinbelow.

There is thus provided the invention in accordance with claim 1. In one example, the pedicle screw is constructed of a titanium alloy and the surface is blasted with calcium phosphate particles.

The surface of the pedicle screw includes crests and valleys of screw threads, a shank of the pedicle screw, a (polyaxial) head of the pedicle screw, or any combination thereof.

The surface of the pedicle screw can be blasted to approximately a 2, 3 or 3.34 µm average roughness.

### DETAILED DESCRIPTION OF EMBODIMENTS

In accordance with an embodiment of the present invention, surfaces of pedicle screws are roughened by using Resorbable Blast Media (RBM), such as by blasting with calcium phosphate. This method creates a textured surface by blasting a machined titanium alloy implant with calcium phosphate particles, which is then passivated, without acid etching, to remove residual media. The surfaces of the pedicle screws which are roughened include the crests and valleys of the screw threads, the screw shank and the screw head (including polyaxial screw heads). Since there is no acid etching, the titanium screw material is not susceptible to titanium grain boundary degradation that can occur during aggressive acid etching.

The following are successful test results of in-vitro and in-vivo testing.

In-Vitro Study:
Several disks (15 mm in diameter and 1 mm thick) made of Ti-6A1-4V were prepared to fit into 24-well plates. Surfaces were either machined to produce 0.2 µm smooth surfaces, or grit blasted to result in 2, 3 or 3.34 µm roughness. Roughness refers to average roughness (RA), which is the average height of the bumps on a surface, measured in micrometers (µm). Surface morphology and chemistry were characterized by scanning electron microscopy (SEM), X-ray photon spectroscopy (XPS) and energy dispersive analysis of X-rays (EDAX). Human osteoblast-like cells were cultured on the disks as well as on tissue cultured treated polystyrene (plastic) until cells reach confluence on plastic. The effect of Ti-6Al-4V surface microstructure on TGF-β1 level of MG63 osteoblast-like cells was also studied. Total and active TGF-β1 levels were measured separately. Data were analyzed by ANOVA (Analysis of Variance) and significant differences between groups determined using the Bonferroni modification of Student's t-test. (p<0.05, v. plastic; p<0.05, v. smooth Ti-6Al-4V; p<0.05, v. Ti-6Al-4V surface with Ra of 3 µm)

Responses of cells to different roughed metal alloy surfaces were analyzed and the results are summarized in the following table:

| Material | ng Osteocalcin/Well | ng Osteocalcin/Cell | ng TGF-β1/Cell |
|---|---|---|---|
| Plastic | <6 | ~3 x 10⁻⁵ | ~3 x 10⁻⁵ |
| Ti-6Al-4V 0.2 µm | <6 | ~5 x 10⁻⁵ | ~3 x 10⁻⁵ |
| Ti-6Al-4V 2 µm | ~7 | ~25 x 10⁻⁵ | ~12 x 10⁻⁵ |
| Ti-6Al-4V 3 µm | ~8 | ~30 x 10⁻⁵ | ~14 x 10⁻⁵ |
| Ti-6Al-4V 3.34 µm | >9 | ~60 x 10⁻⁵ | ~30 x 10⁻⁵ |

It is clear the osteocalcin and Transforming Growth Factor (TGF) level increased in a surface dependent manner, with highest values seen on roughest surfaces.

The results show that roughening of surfaces by RBM promotes the differentiated of osteoblast, inhibit osteoclast cells activity and actively participate in bone remodeling.

In-Vivo Study:
Pedicle screws (ø5 x 25 mm) made of Ti-6Al-4V were implanted into L4 and L5 vertebra of 15 sheep. Fusion rods were connected vertically to attain proper fixation and load bearing. Each sheep received either 4 smooth (Ra=0.2 µm) or 4 rough (Ra=3 µm) screws.

The animals were euthanized after 12 weeks of implantation, the screws in L4 vertebras were sent to histomorphometric analysis to evaluate the Bone-Implant-Contact (BIC) percentage and the screws in L5 vertebras were measured for the required torque to remove them.

The results are summarized in the following table, which shows BIC and removal torque results after 3 months of implantation:

| | Smooth Ra=0.2 µm | Rough Ra=3 µm |
|---|---|---|
| BIC% | 60.29±4.78 | 80.99±4.42 |
| EBIC% | 83.67±1.74 | 81.35±2.96 |
| BV% | 78.07±1.47 | 79.455±1.60 |
| Removal Torque (N-m) | 2.28±0.32 | 5.29±0.41 |

The results of the histomorphological analysis in the above table clearly show that average BIC% around rough surface Ti-6Al-4V implant was significantly higher than that around smooth ones. There were no difference of expected bone to implant contact (EBIC%) and bone volume (BV%) of two groups. The required force to remove screws from the bone showed also significant higher values for rough implants than smooth implants. In short, the roughened surfaces resulted in more than twice removal torque value and a growth of 34% in the bone-implant-contact.

## Claims

1. A method for surface treatment of a pedicle screw comprising:
roughening a surface of a pedicle screw by blasting the surface with a Resorbable Blast Media (RBM), and passivating said surface after RBM blasting without acid etching.

2. The method according to claim 1, comprising blasting said surface with calcium phosphate particles.

3. The method according to claim 1, wherein said pedicle screw is constructed of a titanium alloy and said surface is blasted with calcium phosphate particles.

4. The method according to claim 1, wherein said surface of said pedicle screw comprises at least one of crests and valleys of screw threads.

5. The method according to claim 1, wherein said surface of said pedicle screw comprises at least one of a shank and a head of said pedicle screw.

6. The method according to claim 1, wherein said surface of said pedicle screw comprises a polyaxial head of said pedicle screw.

7. The method according to claim 1, wherein said surface of said pedicle screw is blasted to approximately a 2 µm average roughness.

8. The method according to claim 1, wherein said surface of said pedicle screw is blasted to approximately a 3 µm average roughness.

9. The method according to claim 1, wherein said surface of said pedicle screw is blasted to approximately a 3.34 µm average roughness.

10. An article comprising:
a pedicle screw comprising a surface roughened by blasting the surface with a Resorbable Blast Media (RBM) and passivating said surface after RBM blasting without acid etching.

11. The article according to claim 10, wherein said surface of said pedicle screw has approximately a 3.34 µm average roughness.

## Patentansprüche

1. Ein Verfahren zur Oberflächenbehandlung einer Pedikalschraube umfassend:
Aufrauen einer Oberfläche einer Pedikalschraube durch Strahlen der Oberfläche mit einem resorbierbaren Strahl-Medium (RBM) und Passivieren der Oberfläche nach dem RBM-Strahlen ohne Säureätzen.

2. Das Verfahren gemäß Anspruch 1, umfassend das Strahlen der Oberfläche mit Kalziumphosphatpartikeln.

3. Das Verfahren gemäß Anspruch 1, wobei die Pedikalschraube aus einer Titanlegierung gebildet ist und die Oberfläche mit Kalziumphosphatpartikeln gestrahlt wird.

4. Das Verfahren gemäß Anspruch 1, wobei die Oberfläche der Pedikalschraube Grate und/oder Täler eines Schraubengewindes umfasst.

5. Das Verfahren gemäß Anspruch 1, wobei die Oberfläche der Pedikalschraube einen Schaft und/oder einen Kopf der Pedikalschraube umfasst.

6. Das Verfahren gemäß Anspruch 1, wobei die Oberfläche der Pedikalschraube einen koaxialen Kopf der Pedikalschraube umfasst.

7. Das Verfahren gemäß Anspruch 1, wobei die Oberfläche der Pedikalschraube zu einer durchschnittlichen Rauigkeit von ungefähr 2 µm gestrahlt wird.

8. Das Verfahren gemäß Anspruch 1, wobei die Oberfläche der Pedikalschraube zu einer durchschnittlichen Rauigkeit von ungefähr 3 µm gestrahlt wird.

9. Das Verfahren gemäß Anspruch 1, wobei die Oberfläche der Pedikalschraube zu einer durchschnittlichen Rauigkeit von ungefähr 3,34 µm gestrahlt wird.

10. Ein Artikel umfassend:
eine Pedikalschraube, umfassend eine Oberfläche, welche durch Strahlen mit einem resorbierbaren Strahl-Medium (RBM) aufgeraut ist und nach dem RBM-Strahlen ohne Säureätzen passiviert ist.

11. Der Artikel gemäß Anspruch 10, wobei die Oberfläche der Pedikalschraube eine durchschnittliche Rauigkeit von ungefähr 3,34 µm aufweist.

## Revendications

1. Procédé de traitement superficiel d'une vis pédiculaire, comprenant^ le fait de :
rendre rugueuse une surface d'une vis pédiculaire par projection de particules sur la surface avec un matériau de projection résorbable (RBM) et soumettre ladite surface à une passivation après la projection RBM en l'absence d'un décapage à l'acide.

2. Procédé selon la revendication 1, comprenant le fait de projeter sur ladite surface des particules de phosphate de calcium.

3. Procédé selon la revendication 1, dans lequel ladite vis pédiculaire est réalisée à partir d'un alliage de titane et ladite surface est soumise à une projection avec des particules de phosphate de calcium.

4. Procédé selon la revendication 1, dans lequel ladite surface de ladite vis pédiculaire comprend au moins un membre choisi parmi le groupe comprenant des sommets et des creux de filets de vis.

5. Procédé selon la revendication 1, dans lequel ladite surface de ladite vis pédiculaire comprend au moins un membre choisi parmi le groupe comprenant une queue et une tête de ladite vis pédiculaire.

6. Procédé selon la revendication 1, dans lequel ladite surface de ladite vis pédiculaire comprend une tête multiaxe de ladite vis pédiculaire.

7. Procédé selon la revendication 1, dans lequel ladite surface de ladite vis pédiculaire est soumise à une projection jusqu'à ce que l'on obtienne une rugosité moyenne d'approximativement 2 µm.

8. Procédé selon la revendication 1, dans lequel ladite surface de ladite vis pédiculaire est soumise à une projection jusqu'à ce que l'on obtienne une rugosité moyenne d'approximativement 3 µm.

9. Procédé selon la revendication 1, dans lequel ladite surface de ladite vis pédiculaire est soumise à une projection jusqu'à ce que l'on obtienne une rugosité moyenne d'approximativement 3,34 µm.

10. Article comprenant :
une vis pédiculaire comprenant une surface qui a été rendue rugueuse par projection de particules sur la surface avec un matériau de projection résorbable (RBM) et qui a été soumise à une passivation après la projection RBM en l'absence d'un décapage à l'acide.

11. Article selon la revendication 10, dans lequel dans lequel ladite surface de ladite vis pédiculaire possède une rugosité moyenne d'approximativement 3,34 µm.
